# EUROPEAN PATENT APPLICATION

(11) **EP 1 130 396 A1**
(43) Date of publication of application: **05.09.2001**
(21) Application number: 01301834.6
(22) Date of filing: 28.02.2001
(51) Int. Cl.: G01N 33/53

(54) **Measuring method and measuring reagent of C-reactive protein**

(30) Priority: 29.02.2000 JP 2000054096; 29.02.2000 JP 2000054102
(71) Applicant: KYOWA MEDEX CO., LTD., Tokyo 104-0042 (JP)
(72) Inventor: Yokohama, Hiroaki, Tokyo 158-0083 (JP); Umehara, Harumi, Kyowa Medex Co., Ltd., Res. Lab., Sunto-gun, Shizuoka 411-0932 (JP); Matsumori, Shigeru, Kyowa Medex Co. Ltd., Res.Lab., Sunto-gun, Shizuoka 411-0932 (JP); Yamada, Satoshi, Tsukuba, Ibaraki 305-0045 (JP); Shuto, Kenshiro, Tsukuba, Ibaraki 300-3261 (JP); Sakaki, Shujiro, Tsukuba, Ibaraki 305-0045 (JP); Suzuki, Ken, Tsukuba, Ibaraki 305-0031 (JP)
(74) Representative: Harding, Charles Thomas

(57) **Abstract**

An object of the present invention is to provide a method and a reagent for measuring the subject substances containing high concentration of C-reactive protein without dilution while avoiding prozone phenomenon.

C-reactive protein is measured with a compound having a phosphrylcholine group and a cationic group shown by the general formula (I) [in the formula (I), where R¹, R² and R³ stand for a hydrogen atom, substituted or non-substituted alkyl, or substituted or non-substituted alkenyl, and X⁻ stands for an inorganic anion or an organic anion] and an antibody to C-reactive protein. Or, C-reactive protein is measured with a surface active agent having a phosphorylcholine group, a surface active agent having a cationic group shown by the formura (II) [Y₁ stands for a hydrophobic group, and R₁, R₂ and R₃ stand for a hydrogen atom, substituted or non-substituted alkyl, or substituted or non-substituted alkenyl], and an antibody to C-reactive protein. As an antibody to C-reactive protein, an antibody carried by a water-insoluble carrier such as latex made from polystyrene is preferable.

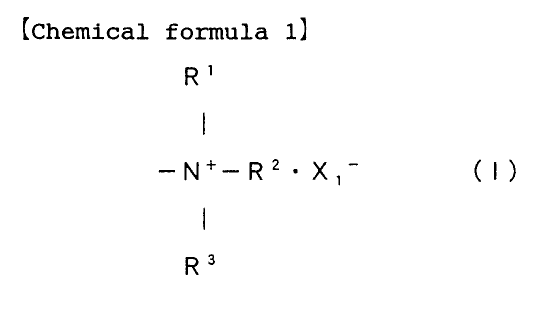

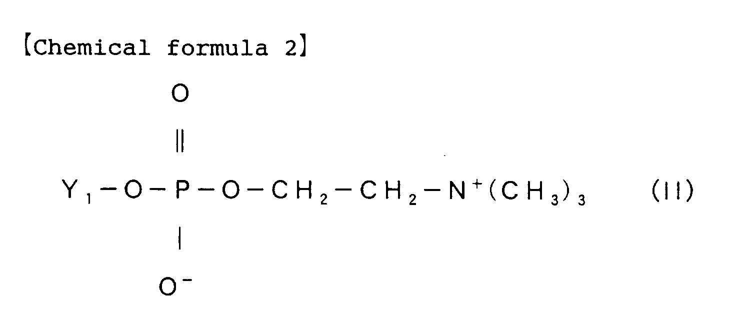

## Description

### Technical Field to which the Invention Pertains:

This invention relates to a measuring reagent and a measuring method of C-reactive protein, in detail, a measuring reagent of C-reactive protein containing a compound having a phosphorylcholine group and a cationic group, and an antibody to C-reactive protein, and a measuring method of C-reactive protein for measuring C-reactive protein with the said reagent, and to a measuring reagent of C-reactive protein containing a surface active agent having a phosphorylcholine group, a surface active agent having a cationic group and an antibody to C-reactive protein, and a measuring method of C-reactive protein for measuring C-reactive protein with the said reagent.

### Prior Art:

C-reactive protein, one of acute phase reaction substances, is used as a marker for diagnosis or process observation of various kinds of infections, inflammatory diseases and diseases that cause the destruction of tissues, and is one of often measured items in the field of clinical examination. Methods using an antibody or antiserum that specifically combines with C-reactive protein, such as a capillary-rise method, one-dimensional immunodiffusion, immunoturbidimetry or latex immunoturbidimetry, are known as measuring methods of such C-reactive protein. These methods make use of the fact that the combination of C-reactive protein, which is an antigen, and an antibody creates large aggregate, and the measurement is conducted by detecting such aggregation.

In latex immunoturbidimetry, for example, when a carrier like polystyrene latex with a particle diameter of about 0.1∼1µm, carrying (sensitized to) an antibody, is used together with its corresponding antigen, and antigen-antibody reaction is caused, the amount of scattered light increases and that of permeated light decreases in the reaction liquid so that C-reactive protein can be measured by detecting the variations as absorbance or as integrating sphere turbidity in the reaction liquid. However, it is known that the problem called prozone phenomenon occurs in these methods of observing aggregation in antigen-antibody reaction. The higher the concentration of an antigen becomes in comparison with that of an antibody, the lower the turbidity becomes conversely; this is called prozone phenomenon. This phenomenon would bring about false results of low concentration of an antigen, in spite that there is high concentration of an antigen in a sample to be measured. In order to avoid this prozone phenomenon, a sample needs to be diluted or to be remeasured after increasing the amount of an antibody used for this measurement, but the operation would be complicated in that case.

It is known that phosphorylcholine specifically combines with C-reactive protein and forms aggregation in the presence of calcium ion [J. Immunol., 124, 1396(1980)]. Also, it is known that C-reactive protein is purified by affinity chromatography using p-nitrophenyl phosphorylcholine Sepharose4B column [Kensa to Gijutsu, 24(5), 409(1996)]. Further, as methods of determining C-reactive protein, a method using a reagent containing phosphatidylcholine, cholesterol, choline chloride, calcium or the like (Japanese Laid-Open Patent Application No.123295/1977), a method using a reagent containing a polymer combined with a phosphorylcholine group, and a method using a reagent containing a polymer combined with a phosphorylcholine group and a specific antibody to C-reactive protein (Japanese Laid-Open Patent Application No. 259063/1987) are known. As a measuring method of C-reactive protein using a latex reagent in which the detection is made by integrating sphere turbidity, for example, a method using a latex reagent of C-reactive protein made by Kyowa Medex Co. Ltd. (Extel CRP for EL-1200) is known.

An object of the present invention is to provide a method of measuring a subject substance containing high concentration of C-reactive protein without dilution while avoiding prozone phenomenon, and a reagent used in the measuring method.

### Disclosure of the Invention:

After intensive study for solving the above-mentioned problem, inventors of the present invention have found that it is possible to measure C-reactive protein in the subject substance, and that even though the subject substance contains high concentration of C-reactive protein, C-reactive protein in the subject substance can be measured without dilution while avoiding prozone phenomenon by using (A) a compound having a phosphorylcholine group and a cationic group, and an antibody to C-reactive protein, or by using (B) a surface active agent having a phosphorylcholine group, a surface active agent having a cationic group, and an antibody to C-reactive protein. The present invention has been thus completed.

In other words, the present invention relates to a measuring method of C-reactive protein characterized in measuring C-reactive protein with a compound having a phosphorylcholine group and a cationic group excluding a phosphorylcholine group, and an antibody to C-reactive protein, or with a surface active agent having a phosphorylcholine group, a surface active agent having a cationic group excluding a phosphorylcholine group, and an antibody to C-reactive protein (claim 1).

The present invention also relates to a measuring method of C-reactive protein according to claim 1, wherein a cationic group in a compound having a phosphorylcholine group and a cationic group excluding a phosphorylcholine group is a group shown by the general formula (I) 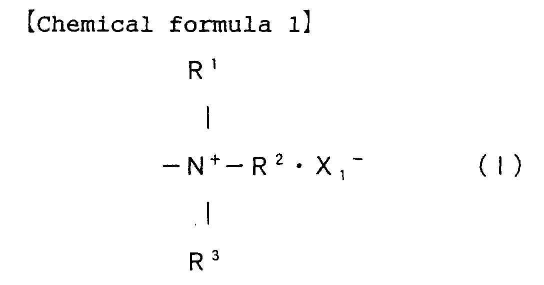 [in the formula (I), R¹, R² and R³ are same or different from one another, each of them stands for a hydrogen atom, substituted or non-substituted alkyl, or substituted or non-substituted alkenyl, and X₁⁻ stands for an inorganic anion or an organic anion](claim 2), a measuring method of C-reactive protein according to claim 1 or 2, wherein a compound having a phosphorylcholine group and a cationic group excluding a phosphorylcholine group is a copolymer created by combining a monomer having a phosphorylcholine group and a monomer having a cationic group (claim 3), a measuring method of C-reactive protein according to claim 3, wherein a monomer having a phosphorylcholine group and a monomer having a cationic group are a monomer having a phosphorylcholine group and a vinyl group, and a monomer having a cationic group and a vinyl group respectively (claim 4), a measuring method of C-reactive protein according to claim 4, wherein a monomer having a phosphorylcholine group and a vinyl group, and a monomer having a cationic group and a vinyl group are 2-methacryloyloxyethyl phosphorylcholine and 2-hydroxy-3-methacryloyloxypropyltrimethylammonium chloride respectively (claim 5) and a measuring method of C-reactive protein according to claim 1, wherein a surface active agent having a phosphorylcholine group is a compound shown by the general formula (II) 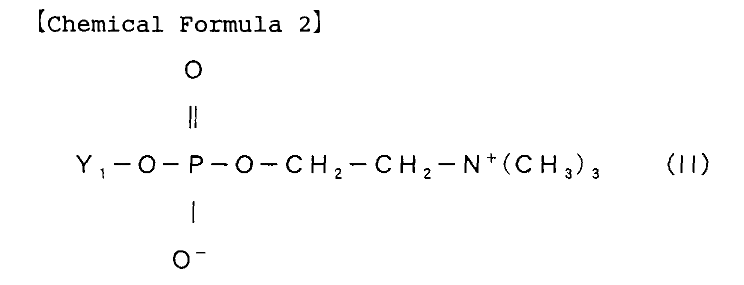 [Y₁ in the formula (II) stands for a hydrophobic group] (claim 6).

The present invention also relates to a measuring method of C-reactive protein according to claim 6, wherein a compound shown by the formula (II) is one or more kinds of compounds selected from lysophosphatidylcholine caproyl, lysophosphatidylcholine myristoyl, lysophosphatidylcholine palmitoyl, lysophosphatidylcholine stearoyl, lysophosphatidylcholine derived from soybeans, phosphatidylcholine dibutyloyl, phosphatidylcholine dicaproyl, phosphorylcholine oleyloxyethyl ester and sphingosyl phosphorylcholine (claim 7), a measuring method of C-reactive protein according to any one of claims 1, 6 or 7, wherein a surface active agent having a cationic group is a surface active agent of ammonium salt (claim 8), a measuring method of C-reactive protein according to claim 8, wherein a surface active agent of ammonium salt is a compound shown by the general formula (III) 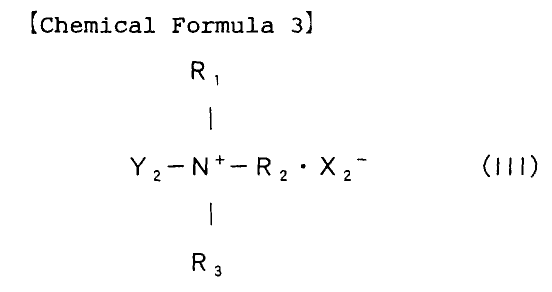 [in the formula (III), Y₂ stands for a hydrophobic group, R₁, R₂ and R₃ are same or different from one another, and each of them stands for a hydrogen atom, substituted or non-substituted alkyl, or substituted or non-substituted alkenyl, and X₂⁻ stands for an inorganic anion or an organic anion] ( claim 9), a measuring method of C-reactive protein according to claim 9, wherein a compound shown by the formula (III) is one or more kinds of compounds selected from octadecyltrimethylammonium chloride, hexadecyltrimethylammonium chloride, tetradecyltrimethylammonium chloride and dodecyltrimethylammonium chloride (claim 10), a measuring method of C-reactive protein according to any one of claims 1∼10, wherein an antibody to C-reactive protein is carried by a water-insoluble carrier (claim 11) and a measuring method of C-reactive protein according to claim 11, wherein a insoluble carrier is latex made from polystyrene (claim 12).

The present invention relates to a measuring reagent of C-reactive protein characterized in containing a compound having a phosphorylcholine group and a cationic group excluding a phosphorylcholine group, and an antibody to C-reactive protein, or containing a surface active agent having a phosphorylcholine group, a surface active agent having a cationic group excluding a phosphorylcholine group, and an antibody to C-reactive protein (claim 13), a measuring reagent of C-reactive protein according to claim 13, wherein a cationic group in a compound having a phosphorylcholine group and a cationic group excluding a phosphorylcholine group is shown by the general formula (I) 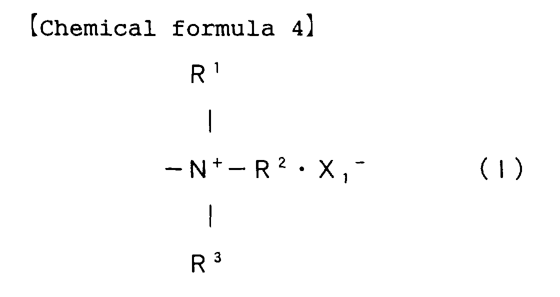 [in the formula (I), R¹, R² and R³ are same or different from one another, and each of them stands for a hydrogen atom, substituted or non-substituted alkyl, or substituted or non-substituted alkenyl, and X₁⁻ stands for an inorganic anion or an organic anion] (claim 14), a measuring reagent of C-reactive protein according to claims 13 or 14, wherein a compound having a phosphorylcholine group and a cationic group excluding a phosphorylcholine group is a copolymer created by combining a monomer having a phosphorylcholine group and a monomer having a cationic group (claim 15), a measuring reagent of C-reactive protein according to claim 15, wherein a monomer having a phosphorylcholine group and a monomer having a cationic group are a monomer having a phosphorylcholine group and a vinyl group, and a monomer having a cationic group and a vinyl group respectively (claim 16) and a measuring reagent of C-reactive protein according to claim 16, wherein a monomer having a phosphorylcholine group and a vinyl group, and a monomer having a cationic group and a vinyl group are 2-methacryloyloxyethyl phosphorylcholine and 2-hydroxy-3-methacryloyloxypropyltrimethylammonium chloride respectively (claim 17) as well.

The present invention also relates to a measuring reagent of C-reactive protein according to claim 13, wherein a surface active agent having a phosphorylcholine group is a compound shown by the general formula (II) 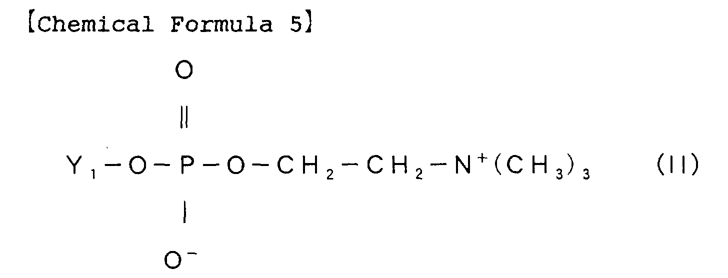 [Y₁ in the formula (II) stands for a hydrophobic group] (claim 18), a measuring reagent of C-reactive protein according to claim 18, wherein a compound shown by the formula (II) is one or more kinds of compounds selected from lysophosphatidylcholine caproyl, lysophosphatidylcholine myristoyl, lysophosphatidylcholine palmitoyl, lysophosphatidylcholine stearoyl, lysophosphatidylcholine derived from soybeans, phosphatidylcholine dibutyloyl, phosphatidylcholine dicaproyl, phosphorylcholine oleyloxyethyl ester and sphingosyl phosphorylcholine (claim 19), a measuring reagent of C-reactive protein according to any one of claims 13, 18 or 19, wherein a surface active agent having a cationic group is a surface active agent of ammonium salt (claim 20), a measuring reagent of C-reactive protein according to claim 20, wherein a surface active agent of ammonium salt is a compound shown by the general formula (III) 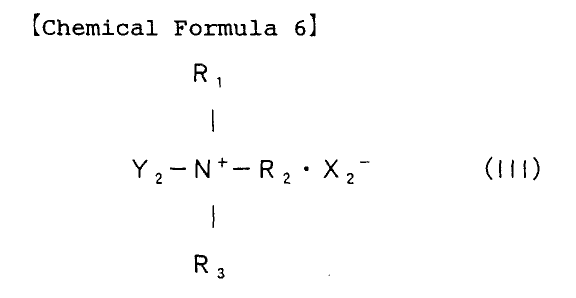 [in the formula (III), Y₂ stands for a hydrophobic group, R₁, R₂ and R₃ are same or different from one another, and each of them stands for a hydrogen atom, substituted or non-substituted alkyl, or substituted or non-substituted alkenyl, and X₂⁻ stands for an inorganic anion or an organic anion] (claim 21), a measuring reagent of C-reactive protein according to claim 21, wherein a compound shown by the formula (III) is one or more kinds of compounds selected from octadecyltrimethylammonium chloride, hexadecyltrimethylammonium chloride, tetradecyltrimethylammonium chloride and dodecyltrimethylammonium chloride (claim 22), a measuring reagent of C-reactive protein according to any one of claims 13∼22, wherein an antibody to C-reactive protein is carried by a water-insoluble carrier (claim 23) and a measuring reagent of C-reactive protein according to claim 23, wherein a insoluble carrier is latex made from polystyrene (claim 24).

### Brief Explanation of Drawings:

Fig. 1 is a view showing the result of measuring 0∼100mg/dL of C-reactive protein with the reagents in example A-1, and comparisons A-1∼A-3.

Fig. 2 is a view showing the result of measuring 0∼100mg/dL of C-reactive protein with the reagents in example A-1, and comparisons A-1 and A-4.

Fig. 3 is a view showing the result of measuring 0∼100mg/dL of C-reactive protein with the reagents in example A-2, and comparison A-5.

Fig. 4 is a view showing the result of measuring 0∼100mg/dL of C-reactive protein with the reagents in example A-3, and comparison A-5.

Fig. 5 is a view showing the result of measuring 0∼100mg/dL of C-reactive protein with the reagents in example A-9, and comparison A-1.

Fig. 6 is a view showing the result of measuring 0∼100mg/dL of C-reactive protein with the reagents in examples B-1 and B-2, and comparisons B-1∼B-4.

Fig. 7 is a view showing the result of measuring 0∼100mg/dL of C-reactive protein with the reagents in example B-3 and comparison B-5.

Fig. 8 is a view showing the result of measuring 0∼100mg/dL of C-reactive protein with the reagents in examples B-2, B-4 and B-5, and comparison B-1.

### Best Mode for Carrying Out the Invention:

The measuring methods of C-reactive protein of the present invention consist of a measuring method characterized in measuring C-reactive protein with a compound having a phosphorylcholine group and a cationic group excluding a phosphorylcholine group, and an antibody to C-reactive protein (hereinafter "the measuring method of C-reactive protein of the present invention (A)"), and a measuring method characterized in measuring C-reactive protein with a surface active agent having a phosphorylcholine group, a surface active agent having a cationic group, and an antibody to C-reactive protein (hereinafter "the measuring method of C-reactive protein of the present invention (B)"), and the measuring reagents of C-reactive protein of the present invention consist of a measuring reagent characterized in containing a compound having a phosphorylcholine group and a cationic group excluding a phosphorylcholine group, and an antibody to C-reactive protein (hereinafter "the measuring reagent of C-reactive protein of the present invention (A)"), and a measuring reagent characterized in containing a surface active agent having a phosphorylcholine group, a surface active agent having a cationic group, and an antibody to C-reactive protein (hereinafter "the measuring reagent of C-reactive protein of the present invention (B)").

As to a compound having a phosphorylcholine group and a cationic group excluding a phosphorylcholine group in the measuring method of C-reactive protein of the present invention (A) and the measuring reagent of C-reactive protein of the present invention (A), though any compounds can be used as long as the compounds have at least one pair of a phosphorylcholine group and a cationic group excluding a phosphorylcholine group in one molecule, the compounds that have more than one pair of a phosphorylcholine group and a cationic group excluding a phosphorylcholine group in one molecule are preferable in consideration of avoiding prozone phenomenon sufficiently. Examples of such compounds are a synthetic compound constructed by induction of these two groups into a natural compound like fat and oil, carbohydrate, protein, polysaccharide or nucleic acid, or a synthetic compound constructed by induction of these two groups into a synthetic compound, or a synthetic compound constructed by combining compounds that have these groups separately through synthesis. The molecular weight of the compounds having a phosphorylcholine group and a cationic group excluding a phosphorylcholine group of the present invention is not limited in particular, but it is preferably 500∼5,000,000, more preferably 1,000∼1,000,000, and most preferably 5,000∼100,000 in consideration of avoiding prozone phenomenon sufficiently.

The cationic group in the above-mentioned compounds having a phosphorylcholine group and a cationic group excluding a phosphorylcholine group is not particularly limited as long as the cationic group has positive charge excluding a phosphorylcholine group, but the cationic group shown by the general formula (I) [in the formula (I), where R¹, R² and R³ are same or different from one another. R¹, R² and R³ stand for a hydrogen atom, substituted or non-substituted alkyl, or substituted or non-substituted alkenyl, and X₁⁻ stands for an inorganic anion or an organic anion] is preferable in consideration of avoiding prozone phenomenon sufficiently.

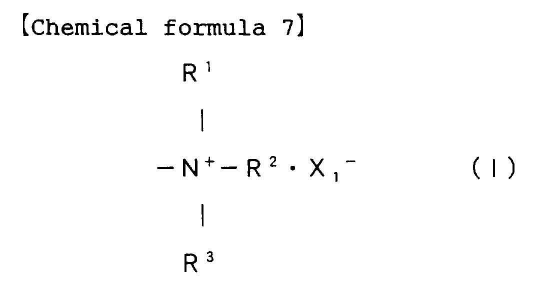

As alkyls in the general formula (I), straight chain or branch chain alkyl, having preferably 1-24 carbon atoms, more preferably 1∼12 carbon atoms, most preferably 1-6 carbon atoms are exemplified, and concrete examples of them include methyl, ethyl, propyl, isopropyl, butyl, isobutyl, sec-butyl, tertbutyl, pentyl, neopentyl, hexyl, heptyl, octyl, nonyl, and decyl. As alkenyls, straight chain or branch chain alkenyl, having preferably 2-24 carbon atoms, more preferably 2-12 carbon atoms, most preferably 2∼6 carbon atoms are exemplified, and concrete examples of them include vinyl, allyl, 2-butenyl, 2-pentenyl, 2-hexenyl. Examples of substituents of substituted alkyl and substituted alkenyl are alkoxy, alkanoyl, alkanoyloxy, alkenyloxy, alkenoyl, alkenoyloxy, aroyl, substituted or non-substituted phenyl and substituted or non-substituted naphtyl. Alkyl moiety of alkoxy, alkanoyl and alkanoyloxy are the same as the said alkyl. Alkenyl moiety of alkenyloxy, alkenoyl and alkenoyloxy are the same as the said alkenyl. Benzoyl and naphthoyl are examples of aroyl. As substituents of substituted phenyl or substituted naphthyl, alkyl, alkenyl, or the likes are exemplified, and the alkyl and the alkenyl are the same as the said alkyl and the said alkenyl.

Halogen such as fluorine, chloride, bromine and iodine, and an inorganic acid anion such as nitric acid are exemplified as examples of an inorganic anion. An organic carboxylic acid ion such as formic acid, acetic acid, or the like is exemplified as the examples of an organic anion.

As a method of inducing a phosphorylcholine group and a cationic group into a natural compound or a synthetic compound, publicly known method, for example, the method of reacting a functional group, such as a hydroxyl group, a carboxyl group and an amino group in a natural compound or a synthetic compound with a functional group being induced to a compound having a phosphorylcholine group and a cationic group by publicly known method is exemplified. As the functional group being induced to a phosphorylcholine group and a cationic group, any functional group that combines with the above mentioned functional group of a natural compound and of a synthetic compound will suffice, and a hydroxyl group, an amino group, a carboxyl group and an aldehyde group and the like are exemplified as those functional groups.

A method of constructing a synthetic compound by combining a compound that has a phosphorylcholine group and a compound that has a cationic group through synthesis is not particularly limited. It is possible to construct a synthetic compound by using a compound having a phosphorylcholine group and a compound having a cationic group as monomers, and then polymerizing these monomers through a method like addition polymerization. Followings are examples of the said monomers; a compound having a phosphorylcholine group and a vinyl group and a compound having a cationic group and a vinyl group; a diol compound having a phosphorylcholine group or a cationic group and a dicalboxylic acid compound having a cationic group or a phosphorylcholine group; a diamine compound having a phosphorylcholine group or a cationic group and a dicalboxylic acid compound having a cationic group or phosphorylcholine group; and, in particular, it is preferable to use the method of constructing a synthetic compound through polymerization of a compound having a phosphorylcholine group and a vinyl group and a compound having a cationic group and a vinyl group in view of controllability of the molecular weight or of the composition ratio.

The above mentioned monomers having a phosphorylcholine group and a vinyl group are not limited in particular, as long as copolymerization of the monomers is possible, and 2-acryloyloxyethyl phosphorylcholine, 2-methacryloyloxyethyl phosphorylcholine (hereinafter abbreviated to MPC), 2-(meth)acryloyl oxyethoxyethyl phosphorylcholine, 6-(meth)acryloyloxyhexyl phosphorylcholine, 10-(meth)acryloyl oxyethoxynonyl phosphorylcholine, allyl phosphorylcholine, butenyl phosphorylcholine, hexenyl phosphorylcholine, octenyl phosphorylcholine, and decenyl phosphorylcholine are the concrete examples of the monomers. Further, these monomers can be constructed by publicly known methods, for instance, by the methods shown in Japanese Laid-Open Patent Application No.6325/1979, and in Japanese Laid-Open Patent Application No.154591/1983.

The above mentioned monomers having a cationic group and a vinyl group are not limited in particular, as long as radical polymerization of the monomers is possible, and [3-(methacryloyl oxyamino) propyl] trimethylammonium chloride, [3-(acryloyl oxyamino) propyl] trimethylammonium chloride, [2-(methacryoyloxy) ethyl] trimethylammonium chloride [2-(acryloyloxy) ethyl] trimethylammonium chloride, 2-hydroxy-3-methacryloyloxypropyltrimethylammonium chloride (hereinafter abbreviated to QA), and 2-hydroxy-3-allyloyloxypropyltrimethylammonium chloride, are exemplified as concrete examples. These monomers are available as general reagents.

The combination of a monomer having a phosphorylcholine group and a vinyl group and a monomer having a cationic group and a vinyl group is not particularly limited, but the combination of the said MPC and QA is preferable in consideration of avoiding prozone phenomenon sufficiently. Besides, in polymerization of a monomer having a phosphorylcholine group and a vinyl group and a monomer having a cationic group and a vinyl group, it is possible to use other radically polymerizable monomers having a vinyl group mixed with the aforementioned monomers. Examples of the said radically polymerizable monomers having a vinyl group are; ester (meth)acrylate including methyl (meth)acrylate, ethyl (meth)acrylate, n-butyl (meth)acrylate,isobutyl(meth)acrylate,pentyl(meth)acrylate, hexyl (meth)acrylate, heptyl (meth)acrylate, octyl (meth)acrylate, tridecyl (meth)acrylate and 2-hydroxyethyl methacrylate; monomers made from styrene including styrene, α -methylstyrene, methyl nuclei-substituted styrene and chloro-substituted styrene; monomers made from substituted or non-substituted carbohydrate including vinyl chloride, vinylidene chloride, ethylene, propylene, isobutylene; monomers made from vinyl ether including ethyl vinyl ether, n-butyl vinyl ether.

As the methods of polymerizing compounds that contain monomers having a phosphorylcholine group and a vinyl group, monomers having a cationic group and a vinyl group used in the present invention, and other radically polymerizable monomers having a vinyl group which is used on demand, publicly known methods such as methods shown in Japanese Laid-Open Patent Application No.3132/1997, Japanese Laid-Open Patent Application No.333421/1996, Japanese Laid-Open Patent Application No.35605/1999 are exemplified. Concretely, the polymerization is possible by radical polymerization under the condition of polymerization temperature of 30∼150°C, and of polymerization hours of 2∼72 hours. As initiators of radical polymerization reaction, 2,2'-azobis (2-methylpropiono amidine) dihydrochloride, 4,4'-azobis (4-cyanovaleric acid), 2,2'-azobis [2-(5-methyl-2-imidazoline-2-il) propane] dihydrochloride, 2,2'-azobisisobutylamide dihydrate, 2,2'-azobisisobutyronitrile, ammonium persulfate, potassium persulfate, benzoyl peroxide, diisopropylperoxy dicarbonate, tert-butylperoxy-2-ethylhexanoate, tert-butylperoxy pivalate, tert-butylperoxy diisobutylate, lauroyl peroxide, azobisisobutyronitrile, 2,2'-azobis (2,4-dimethylvaleronitrile), tert-butylperoxy neodecanoate, and their mixtures are exemplified. As polymerization solvents, water, ethanol, methanol, isopropanol, tert-butanol, benzene, toluene, dimethylformamide, tetrahydrofuran, chloroform, and their mixtures are exemplified.

In polymerization of the said MPC and QA, it is preferable to use 2,2'-azobis (2-methylpropiono amidine) dihydrochloride as an polymerization initiator in consideration of polymerizability or the like, and as to the amount of use, it is preferably 0.01∼10 part by weight, more preferably 0.1∼5 part by weight per 100 part by weight of the ingredients of the monomer. It is particularly preferable to use water or ethanol as polymerization solvent of MPC and QA in consideration of solubility and polymerizability. Polymers can be purified by general purifying methods such as a reprecipitation method, a dialysis method, or an ultrafiltration method.

The molecular weight of a polymer used in the present invention is not particularly limited, but preferably 500∼5,000,000, more preferably 1,000∼1,000,000, most preferably 5,000-100,000. Gel permeation chromatography (GPC) is used to determine this molecular weight with poly(ethylene oxide) standards. Mole fraction of a cationic group in a copolymer that contains a monomer having a phosphorylcholine group and a monomer having a cationic group as a unit is preferably 1∼95%, more preferably 5∼90%, and most preferably 10∼30% to a phosphorylcholine group.

The measurement of C-reactive protein with the measuring method of C-reactive protein of the present invention (A) and with the measuring reagent of C-reactive protein of the present invention (A) is conducted by bringing a compound having a phosphorylcholine group and a cationic group and an antibody to C-reactive protein into contact with a subject substance containing C-reactive protein in the reaction liquid. The reaction liquid is not limited in particular, as long as it is aqueous medium, but buffer liquid is preferable. Glycine buffer, Tris buffer, phosphoric acid buffer and HEPES buffer are examples of such buffer liquid. Further, it is preferable to add calcium ions like calcium chloride to the reaction liquid because this addition enhances the effect of avoiding prozone phenomenon and broadens the range of measurement. The concentration of calcium ion is preferably 1∼20 mmol/L, and more preferably 2∼10 mmol/L. The concentration of compounds having a phosphorylcholine group and a cationic group in the reaction liquid of measurement of C-reactive protein is not particularly limited, but preferably 0.0001∼1 weight %, more preferably 0.005∼0.5 weight %, and most preferably 0.01∼0.1 weight %. Polymers having a phosphorylcholine group and a cationic group have an effect on avoiding prozone phenomenon and broadening the range of measurement even when the added amount is relatively little as above-mentioned.

As the surface active agent having a phosphorylcholine group in the measuring method of C-reactive protein of the present invention (B) and in the measuring reagent of C-reactive protein of the present invention (B), any substance that has a phosphorylcholine group and shows surface activity can be used, but it is preferable to use the surface active agent shown by the general formula (II) [Y₁ in the formula (II) stands for a hydrophobic group] in consideration of avoiding prozone phenomenon sufficiently.

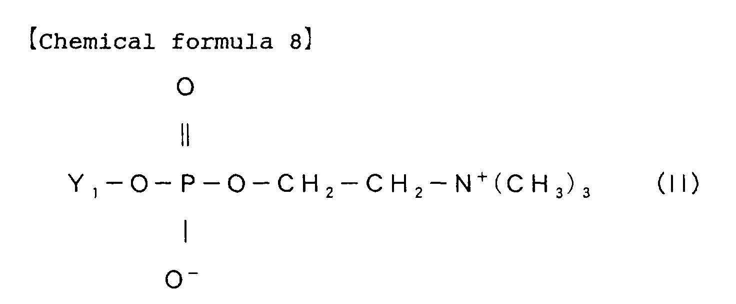

A hydrophobic group in the surface active agent shown by the general formula (II) is not particularly limited as long as it shows the hydrophobicity unlike the hydrophilicity of a phosphorylcholine group, but groups that have hydrocarbon as basic structure, more concretely, substituted or non-substituted alkyl, or substituted or non-substituted alkenyl are exemplified as examples. As alkyls in the general formula (II), straight chain or branch chain alkyl, having preferably 1∼30 carbon atoms, more preferably 2∼24 carbon atoms, most preferably 3∼20 carbon atoms are exemplified, and concrete examples include methyl, ethyl, propyl, isopropyl, butyl, isobutyl, sec-butyl, tert-butyl, pentyl, neopentyl, hexyl, heptyl, octyl, nonyl, decyl, dodecyl, pentadecyl, and eicosyl. As alkenyls, straight chain or branch chain alkenyl, having preferably 2∼24 carbon atoms, more preferably 3∼12 carbon atoms, most preferably 3∼6 carbon atoms are exemplified, and concrete examples of them include vinyl, allyl, 2-butenyl, 2-pentenyl, and 2-hexenyl. Examples of substituents of substituted alkyl and substituted alkenyl are alkoxy, alkanoyl, alkanoyloxy, alkenyloxy, alkenoyl, alkenoyloxy, aroyl, hydroxy, substituted or non-substituted amino, substituted or non-substituted phenyl and substituted or non-substituted naphtyl. Alkyl moiety of alkoxy, alkanoyl and alkanoyloxy are the same as the said alkyl. Alkenyl moiety of alkenyloxy, alkenoyl and alkenoyloxy are the same as the said alkenyl. Benzoyl, naphthoyl, and the likes are examples of aroyl. As substituents of substituted amino, alkyl, alkenyl, and the likes are exemplified, and as substituents of substituted phenyl or substituted naphthyl, hydroxy, alkyl, alkenyl, and the likes are exemplified, and the alkyl and the alkenyl mentioned here are the same as the said alkyl and the said alkenyl.

As the surface active agent shown by the said general formula (II) of the present invention, the surface active agent having alkyl or alkenyl having 2∼24 carbon atoms (including the case that there are oxymethylenyl, oxyethylrenyl, oxypropylrenyl between a phosphorylcholine group and the said alkyl or the said alkenyl), 1- or 2-monoglyceride having 4∼24 carbon atoms in its acyl chain, diglyceride having 4∼24 carbon atoms in its same or different acyl chain, or sphingosine structure as a hydrophobic group is preferable, and lysophosphatidylcholine (lysolecithin) such as lysophosphatidylcholine caproyl, lysophosphatidylcholine myristoyl, lysophosphatidylcholine palmitoyl, lysophosphatidylcholine stearoyl and lysophosphatidylcholine derived from soybeans; phosphatidylcholine of short acyl chain such as phosphatidylcholine dibutyloyl and phosphatidylcholine dicaproyl; and phosphorylcholine oleyloxyethyl ester and sphingosyl phosphorylcholine are concretely exemplified.

As the surface active agent having a cationic group in the measuring method of C-reactive protein of the present invention (B) and in the measuring reagent of C-reactive protein of the present invention (B), any substance that has a cationic group excluding a phosphorylcholine group and shows surface activity can be used, but it is preferable to use the surface active agent of ammonium salt, and the surface active agent shown by the general formula (III) [in the formula (III), Y₂ stands for a hydrophobic group, R₁, R₂ and R₃ are same or different from one another, and each of them stands for a hydrogen atom, substituted or non-substituted alkyl, or substituted or non-substituted alkenyl, and X₂⁻ stands for an inorganic anion or an organic anion] is most preferable in consideration of avoiding prozone phenomenon sufficiently.

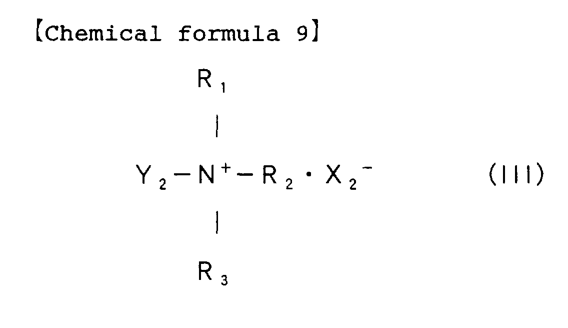

The hydrophobic group Y₂ in the general formula (III) is not limited particularly as long as it shows hydrophobicity unlike the hydrophilicity of a cationic group, but groups that have hydrocarbon as basic structure, more concretely, substituted or non-substituted alkyl, or substituted or non-substituted alkenyl are exemplified as examples. As the above mentioned alkyls, straight chain or branch chain alkyl, having preferably 1∼30 carbon atoms, more preferably 2∼24 carbon atoms, most preferably 3∼20 carbon atoms are exemplified, and concrete examples of them include methyl, ethyl, propyl, isopropyl, butyl, isobutyl, sec-butyl, tert-butyl, pentyl, neopentyl, hexyl, heptyl, octyl, nonyl, decyl, dodecyl, pentadecyl and eicosyl. As alkenyls, straight chain or branch chain alkenyl, having preferably 2∼24 carbon atoms, more preferably 3∼12 carbon atoms, most preferably 3∼6 carbon atoms are exemplified, and concrete examples of them include vinyl, allyl, 2-butenyl, 2-pentenyl and 2-hexenyl. Examples of substituents of substituted alkyl and substituted alkenyl, alkoxy, alkanoyl, alkanoyloxy, alkenyloxy, alkenoyl, alkenoyloxy, aroyl, hydroxy, substituted or non-substituted amino, substituted or non-substituted phenyl and substituted or non-substituted naphtyl are exemplified. Alkyl moiety of alkoxy, alkanoyl and alkanoyloxy are the same as the said alkyl. Alkenyl moiety of alkenyloxy, alkenoyl and alkenoyloxy are the same as the said alkenyl. Benzoyl, naphthoyl, and the likes are examples of aroyl. As substituents of substituted amino, alkyl, alkenyl, and the likes are exemplified, and as substituents of substituted phenyl or substituted naphthyl, hydroxy, alkyl, alkenyl, and the likes are exemplified, and the alkyl and the alkenyl mentined here are the same as the said alkyl and the said alkenyl.

As alkyls in R₁, R₂ and R₃, straight chain or branch chain alkyl, having preferably 1∼12 carbon atoms, more preferably 1∼6 carbon atoms are exemplified, and concrete examples of them include methyl, ethyl, propyl, isopropyl, butyl, isobutyl, sec-butyl, tert-butyl, pentyl, neopentyl, hexyl, heptyl, octyl, nonyl and decyl. As alkenyls in R₁, R₂ and R₃, straight chain or branch chain alkenyl, having preferably 2-12 carbon atoms, more preferably 3∼6 carbon atoms are exemplified, and concrete examples of them include vinyl, allyl, 2-butenyl, 2-pentenyl and 2-hexenyl. Examples of substituents of substituted alkyl and substituted alkenyl in R₁, R₂ and R₃ are alkoxy, alkanoyl, alkanoyloxy, alkenyloxy, alkenoyl, alkenoyloxy, aroyl, hydroxy, substituted or non-substituted amino, substituted or non-substituted phenyl and substituted or non-substituted naphtyl. Alkyl moiety of alkoxy, alkanoyl and alkanoyloxy are the same as the said alkyl in R₁, R₂ and R₃. Alkenyl moiety of alkenyloxy, alkenoyl and alkenoyloxy are the same as the said alkenyl in R₁, R₂ and R₃. Benzoyl and naphthoyl are examples of aroyl. As substituents of substituted amino, alkyl, alkenyl, and the likes are exemplified, and as substituents of substituted phenyl or substituted naphthyl, hydroxy, alkyl, alkenyl, and the likes are exemplified, and the alkyl and the alkenyl mentioned here are the same as the said alkyl and the said alkenyl.

Halogen such as fluorine, chloride, bromine and iodine, and an inorganic acid anion such as nitric acid are exemplified as examples of an inorganic anion. An organic carboxylic acid ion such as formic acid and acetic acid are exemplified as the examples of an organic anion.

As a surface active agent having the above mentioned cationic group, long chain alkyl ammonium salt such as octadecyltrimethylammonium chloride, hexadecyltrimethylammonium chloride, tetradodecyltrimethylammonium chloride and dodecyltrimethylammonium chloride; and long chain alkyl amine salt such as hexadecylamine acetate, octadecylamine hydrochloride, alkylpyridinium salt, and the likes are concretely exemplified.

Though a surface active agent having a phosphorylcholine group and a surface active agent having a cationic group are combined discretionally, it is preferable to use a surface active agents having a phosphorylcholine group and a surface active agent having a cationic group that have an approximately same chain length of a hydrophobic group in consideration of avoiding prozone phenomenon sufficiently. For instance, combination of lysophosphatidylcholine palmitoyl having 16 carbon atoms in its acyl chain as an example of the former one and hexadecyltrimethylammonium chloride having 16 carbon atoms in its alkyl chain as an example of the latter one, or that of lysophosphatidylcholine stearoyl having 18 carbon atoms in its acyl chain as an example of the former one and octadecyltrimethylammonium chloride having 18 carbon atoms in its alkyl chain as an example of the latter one is preferable.

As to the amount of a surface active agent having a phosphorylcholine group and that of a surface active agent having a cationic group, it is desirable to use the amount so that the concentration of each surface active agent in the reaction liquid for measuring C-reactive protein would be 0.0001∼5 weight %, preferably 0.001∼1 weight %, more preferably 0.01∼0.5 weight %. Further, the molar ratio of a surface active agent having a phosphorylcholine group to a surface active agent having a cationic group is at discretion, but it is preferably about 1:10∼5:10.

Either of a polyclonal antibody or a monoclonal antibody will do as the antibody to C-reactive protein of the present invention, and an antibody on the market or an antibody prepared by publicly known methods can be used as these antibodies. It is preferable to use an antibody carried by (sensitized with) a water-insoluble carrier as an antibody to C-reactive protein. Latex, particularly latex made from polystyrene is preferable as a water-insoluble carrier because it is easy to make the carrier carry an antibody. The preferable particle diameter of the said water-insoluble carrier is 0.1∼1µm. An antibody is loaded onto a water-insoluble carrier by publicly known sensitization methods. The concentration of an antibody used here is not limited particularly, but a certain concentration that enables C-reactive protein to be measured only by the antibody is preferable.

The measuring method of C-reactive protein of the present invention (A) is the measuring method making use of antigen-antibody reaction characterized in measuring C-reactive protein with a compound having a phosphorylcholine group and a cationic group, and an antibody to C-reactive protein, and the measuring method of C-reactive protein of the present invention (B) is the measuring method making use of antigen-antibody reaction characterized in measuring C-reactive protein with a surface active agent having a phosphorylcholine group, a surface active agent having a cationic group, and an antibody to C-reactive protein. As these measuring methods making use of antigen-antibody reaction, any measuring methods such as publicly known immunoturbidimetry, latex immunoturbidimetry, gelatin aggregation reaction, liposome immunoassay, fluoroimmunoassay, enzyme immunoassay can be used, but latex immunoturbidimetry is preferable in consideration of avoiding prozone phenomenon sufficiently. As latex, latex made from polystyrene is preferable, and as a particle diameter of the said latex, 0.1∼1µm is preferable. In these methods, a prescribed amount of antibody sensitized latex suspension, a prescribed amount of buffer liquid, and standard liquid with known concentration or a certain amount of the subject substance are mixed and then stirred enough, and the variations of integrating sphere turbidity (ΔIST value) is measured through the variations of turbidity at designated intervals with an integrating sphere turbidimetry, or the variations of absorbance (Δ mAbs. value) is measured at designated intervals with an absorption spectro photometer.

The measuring reagent of C-reactive protein of the present invention (A) is characterized in containing a compound having a phosphorylcholine group and a cationic group and an antibody to C-reactive protein, and the measuring reagent of C-reactive protein of the present invention (B) is characterized in containing a surface active agent having a phosphorylcholine group, a surface active agent having a cationic group, and an antibody to C-reactive protein. In addition to the above mentioned compound and an antibody, these reagents may contain various kinds of surface active agents, inorganic salts, buffers or the like. Triton X-100, Tween 20, and the likes as surface active agents, calcium salt like calcium chloride as inorganic salts, glycine buffer, Tris buffer, phosphoric acid buffer, HEPES buffer, and the likes as buffer liquids are exemplified respectively. As to the amount of a surface active agent, inorganic salt and buffer liquid contained in the measuring reagent of C-reactive protein, it is desirable to use the amount so that the concentration of the substances in the reaction liquid would be 0.001∼0.1 weight %, in particular, 1∼7mmol/L, 0.1∼10mmol/L and 10∼200mmol/L respectively.

As aforementioned, in the measurement of C-reactive protein with the measuring method of C-reactive protein and the measuring reagent of C-reactive protein of the present invention, C-reactive protein is measured with an antibody to C-reactive protein, and a measuring method of an antibody to C-reactive protein and a measuring reagent of an antibody to C-reactive protein can be provided by substituting an antibody with an antigen in the measuring method of C-reactive protein and in the measuring reagent of C-reactive protein, in other words, by substituting an antibody to C-reactive protein with an antigen for C-reactive protein. C-reactive protein itself or the peptide containing phosphorylcholine binding site of C-reactive protein and epitope of anti-C-reactive protein is an example of the said antigen to C-reactive protein. Further, it is preferable to use the said antigen to C-reactive protein after sensitizing it with a carrier such as polystyrene latex with a particle diameter of about 0.1∼1µm, same as an antibody to C-reactive protein.

With regards to (a) the measuring method of an antibody to C-reactive protein with a compound having a phosphorylcholine group and a cationic group, and an antigen to C-reactive protein, or the measuring reagent of an antibody to C-reactive protein containing a compound having a phosphorylcholine group and a cationic group, and an antigen for C-reactive protein, or to (b) the measuring method of an antibody to C-reactive protein with a surface active agent having a phosphorylcholine group, a surface active agent having a cationic group, and an antigen for C-reactive protein, or the measuring reagent of an antibody to C-reactive protein containing a surface active agent having a phosphorylcholine group, a surface active agent having a cationic group and an antigen for C-reactive protein, like the cases of the measuring method of C-reactive protein and the measuring reagent of C-reactive protein of the present invention, it would be possible to avoid prozone phenomenon and to broaden the measurement range, and to determine the subject substance without dilution even if the subject substance contains high concentration of an antibody to C-reactive protein.

The present invention will be explained in detail with examples, comparisons and references, but the technical scope of the present invention is not limited to these examples and the like.

### Example A-1

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 4 (hereinafter described) | 20mg/L |

| Reagent 2 | |
|---|---|
| Anti-C-reactive protein antibody sensitized latex (prepared by reference 1 (hereinafter described)) | 1g/L |

### Comparison A-1

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |

| Reagent 2 | |
|---|---|
| Anti-C-reactive protein antibody sensitized latex (prepared by reference 1) | 1g/L |

### Comparison A-2

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 6 (hereinafter described) | 20mg/L |

| Reagent 2 | |
|---|---|
| Anti-C-reactive protein antibody sensitized latex (prepared by reference 1) | 1g/L |

### Comparison A-3

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 7 (hereinafter described) | 20mg/L |

| Reagent 2 | |
|---|---|
| Anti-C-reactive protein antibody sensitized latex (prepared by reference 1) | 1g/L |

### Comparison A-4

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 4 | 20mg/L |

| Reagent 2 | |
|---|---|
| Anti-C-reactive protein antibody non-sensitized (prepared by reference 2 (hereinafter described)) | latex 1g/L |

### Example A-2

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 5 (hereinafter described) | 30mg/L |

| Reagent 2 | |
|---|---|
| Anti-C-reactive protein antibody sensitized latex (prepared by | |
| reference 3 (hereinafter described)) | 1g/L |

### Comparison A-5

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |

| Reagent 2 | |
|---|---|
| Anti-C-reactive protein antibody sensitized latex (prepared by reference 3) | 1g/L |

### Example A-3

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 4 | 15mg/L |

| Reagent 2 | |
|---|---|
| Anti-C-reactive protein antibody sensitized latex (prepared by reference 3) | 1g/L |

### Example A-4

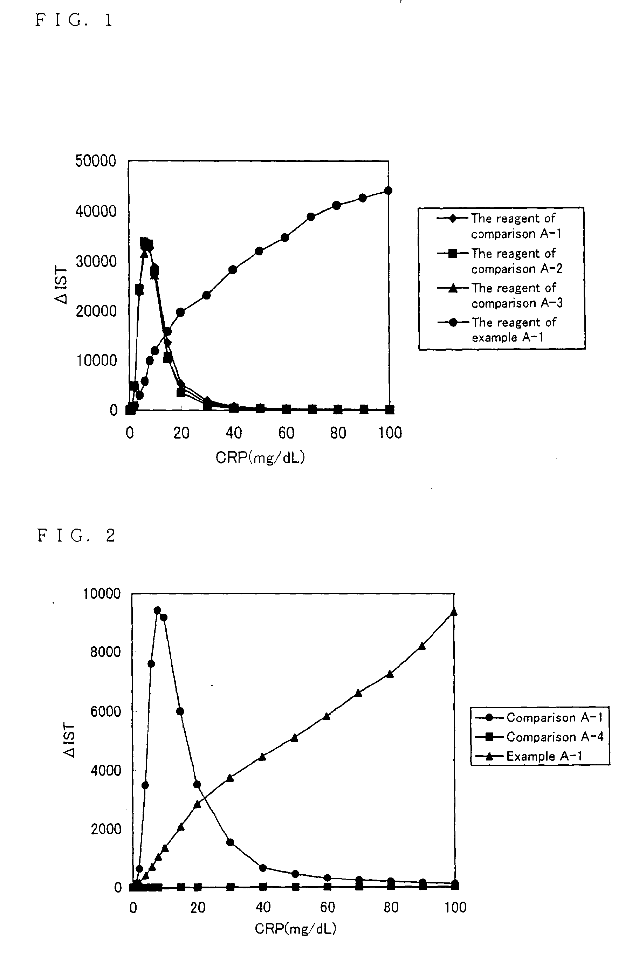

The standard serums containing C-reactive protein of various kinds of concentration (0∼100mg/dL) were measured by EL-1060 (made by Kyowa Medex Co., Ltd.), an equipment only for measuring integrating sphere turbidity, with the latex reagents of measuring C-reactive protein prepared by example A-1 and comparisons A-1∼A-3. 148µL of the reagent 1, 150µL of the reagent 2 and 2µL of the standard serum containing C-reactive protein in example A-1 and comparisons A-1∼A-3 respectively were mixed at 37°C, then the variations of integrating sphere turbidity between 72 seconds and 612 seconds (23∼53 points) were measured. The results are shown in Fig. 1. In Fig. 1, CRP of the horizontal axis stands for the concentration of C-reactive protein, and △IST value of the vertical axis stands for the variations of integrating sphere turbidity.

As Fig. 1 shows, in the measurement with the reagent of comparison A-1 (-◆-plot), with the reagent of comparison A-2 (-■-plot), and with the reagent of comparison A-3 (-▲-), prozone occurred when the concentration of C-reactive protein was 10mg/dL or over, and there was no effect of avoiding prozone. On the contrary, in the measurement with the reagent of example A-1 (-●-), prozone could be avoided at least up to the concentration of 100mg/dL. Prozone could not be avoided even when using the reagent prepared by changing the concentration of the polymer of the reagent 1 in comparison A-2 to the prescribed concentration between 10mg/L∼1g/L, and when using the reagent prepared by changing the concentration of the polymer of the reagent 1 in comparison A-3 to the prescribed concentration between 10mg/L∼1g/L. It has been confirmed that prozone could be avoided with the reagent prepared by changing the concentration of the polymer of the reagent 1 in example A-1 to the concentration of 5mg/L.

### Example A-5

The standard serums containing C-reactive protein of various kinds of concentration (0∼100mg/dL) were measured by EL-1060, an equipment only for measuring integrating sphere turbidity, with the latex reagents of measuring C-reactive protein prepared by example A-1 and comparisons A-1 and A-4. 148µL of the reagent 1, 150µL of the reagent 2 and 2µL of the standard serum containing C-reactive protein in example A-1 and comparisons A-1 and A-4 respectively were mixed at 37°C, then the variations of integrating sphere turbidity between 72 seconds and 216 seconds (23-31 points) were measured. The results are shown in Fig. 2. In Fig. 2, CRP of the horizontal axis stands for the concentration of C-reactive protein, and △IST value of the vertical axis stands for the variations of integrating sphere turbidity.

As Fig. 2 shows, in the measurement with the reagent of comparison A-1 (-●-plot), prozone occurred and ΔIST value decreased when the concentration of C-reactive protein was 10mg/dL or over. In the measurement with the reagent of comparison A-4 (-■-plot), in which only latex non-sensitized with an antibody was used, increase of ΔIST value was not observed. In the measurement with the reagent of example A-1 (-▲-plot), prozone phenomenon was not observed at least up to the C-reactive protein concentration of 100mg/dL and △IST value increased concentration-dependently and the range of measurement was broaden. Considering these results, it is presumed that prozone phenomenon could be avoided in the measuring method of the present invention not by the mutually additive effect of increases of turbidity; one of the said increase was caused by reaction between a compound having a phosphorylcholine group and a cationic group and C-reactive protein, and the other was caused by reaction between an antibody to C-reactive protein and C-reactive protein, but by the synergistic effect of a compound having a phosphorylcholine group and a cationic group, and an antibody to C-reactive protein in the reaction with C-reactive protein.

### Example A-6

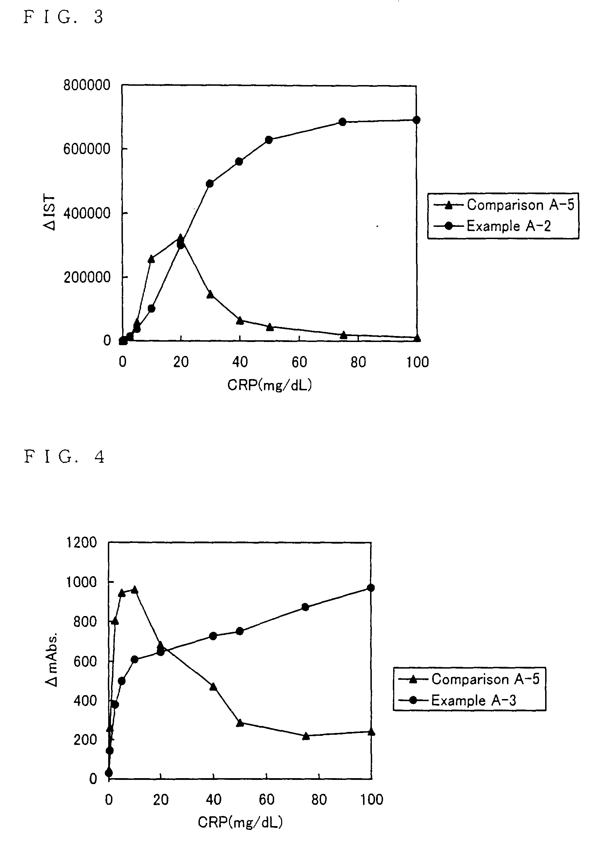

The standard serums containing C-reactive protein of various kinds of concentration (0∼100mg/dL) were measured by EL-1200 (made by Kyowa Medex Co., Ltd.), an equipment only for measuring integrating sphere turbidity, with the latex reagents of measuring C-reactive protein prepared by example A-2 and comparison A-5. 248µL of the reagent 1, 250µL of the reagent 2 and 2µL of the standard serum containing C-reactive protein in example A-2 and comparison A-5 respectively were mixed at 37°C, then the variations of integrating sphere turbidity between 108 seconds and 270 seconds (6∼15 points) were measured. The results are shown in Fig. 3. In Fig. 3, CRP of the horizontal axis stands for the concentration of C-reactive protein, and ΔIST value of the vertical axis stands for the variations of integrating sphere turbidity.

As Fig. 3 shows, in the measurement with the reagent of comparison A-5 (-▲-), Δ IST value decreased by prozone phenomenon when the concentration of C-reactive protein was 20mg/dL or over. Whereas in the measurement with the reagent of example A-2 (-●-), prozone phenomenon could be avoided up to the concentration of 100mg/dL, and quantitativity improved greatly. A same effect was observed when using the reagent prepared by changing the concentration of the polymer in the reagent 1 of example A-2 to the concentration of 10mg/L.

### Example A-7

The standard serums containing C-reactive protein of various kinds of concentration (0∼100mg/dL) were measured by absorption spectro photometric autoanalyzer 7070 (made by Hitachi Ltd.) with the latex reagents of measuring C-reactive protein prepared by example A-3 and comparison A-5.

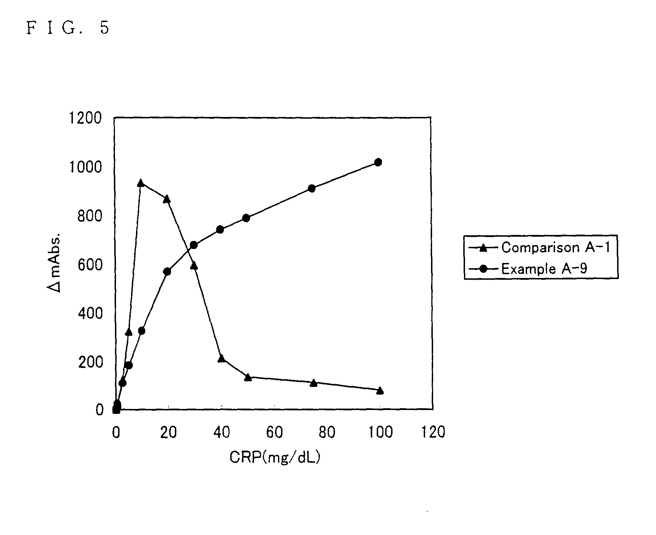

225µL of the reagent 1, 75µL of the reagent 2 and 3µL of the standard serum containing C-reactive protein in example A-3 and comparison A-5 respectively were mixed at 37°C, then the variations of absorbance (wave-length 570nm) between 19 points and 27 points were measured. The results are shown in Fig. 4. In Fig. 4, CRP of the horizontal axis stands for the concentration of C-reactive protein, and ΔmAbs. of the vertical axis stands for the variations of absorbance. As Fig. 4 shows, in the measurement with the reagent of comparison A-5 (-▲-), prozone occurred when the concentration of C-reactive protein was 10mg/dL or over, and the measured value decreased. Whereas in the measurement with the reagent of example A-3 (-●-), prozone could be avoided at least up to the concentration of 100mg/dL, and the quantitativity improved greatly.

### Example A-8

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Polymer made by reference 5 | 20mg/L |

| Reagent 2 | |
|---|---|
| Anti-C-reactive protein antibody sensitized latex (prepared by reference 1) | 1g/L |

### Example A-9

The following measuring reagents of C-reactive protein are prepared.

| Reagent 1 | |
|---|---|
| Glycine buffer, pH8.6 (made by Kanto Chemical Co., Inc.) | 100mmol/L |
| Calcium chloride (made by Kanto Chemical Co., Inc.) | 5mmol/L |
| Ingredient with molecular weight of 10,000 or under prepared by reference 8 (hereinafter described) | 20mg/L |

| Reagent 2 | |
|---|---|
| Anti-C-reactive protein antibody sensitized latex (prepared by reference 1) | 1g/L |

### Example A-10

The standard serums containing C-reactive protein of various kinds of concentration (0∼100mg/dL) were measured by absorption spectro photometric autoanalyzer 7070 (made by Hitachi Ltd.) with the latex reagents of measuring C-reactive protein prepared by example A-9 and comparison A-1. 225µL of the reagent 1, 75µL of the reagent 2 and 3µL of the standard serum containing C-reactive protein in example A-9 and comparison A-1 respectively were mixed at 37°C, then the variations of absorbance (wave-length 570nm) between 19 points and 27 points were measured. The results are shown in Fig. 5. In Fig. 5, CRP of the horizontal axis stands for the concentration of C-reactive protein, and ΔmAbs. of the vertical axis stands for the variations of absorbance. As Fig. 5 shows, in the measurement with the reagent of comparison A-1 (-▲-), prozone occurred when the concentration of C-reactive protein was 10mg/dL or over, and the measured value decreased. Whereas in the measurement with the reagent of example A-9 (-●-), prozone could be avoided at least up to the concentration of 100mg/dL, and the quantitativity improved greatly.

### Example B-1

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Hexadecyltrimethylammonium chloride (NISSAN CationPB-40, made by NOF Corporation) | 0.5g/L |
| Lysolecithin (made by Sigma Chemical Co.) | 0.1g/L |

| Reagent 2 | |
|---|---|
| Anti-C-reactive protein antibody sensitized latex (prepared by reference 1) | 1g/L |

### Example B-2

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Hexadecyltrimethylammonium chloride (NISSAN CationPB-40, made by NOF Corporation) | 0.5g/L |
| Lysolecithin (made by Sigma Chemical Co.) | 0.2g/L |

| Reagent 2 | |
|---|---|
| Anti-C-reactive protein antibody sensitized latex (prepared by reference 1) | 1g/L |

### Comparison B-1

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |

| Reagent 2 | |
|---|---|
| Anti-C-reactive protein antibody sensitized latex (prepared by reference 1) | 1g/L |

### Comparison B-2

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Hexadecyltrimethylammonium chloride (NISSAN CationPB-40, made by NOF Corporation) | 0.5g/L |

| Reagent 2 | |
|---|---|
| Anti-C-reactive protein antibody sensitized latex (prepared by reference 1) | 1g/L |

### Comparison B-3

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Lysolecithin (made by Sigma Chemical Co.) | 0.2g/L |

| Reagent 2 | |
|---|---|
| Anti-C-reactive protein antibody sensitized latex (prepared by reference 1) | 1g/L |

### Comparison B-4

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Hexadecyltrimethylammonium chloride (NISSAN CationPB-40, made by NOF Corporation) | 0.5g/L |
| Lysolecithin (made by Sigma Chemical Co.) | 0.1g/L |

| Reagent 2 | |
|---|---|
| Anti-C-reactive protein antibody non-sensitized latex (prepared by reference 2) | 1g/L |

### Example B-3

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Hexadecyltrimethylammonium chloride (NISSAN CationPB-40, made by NOF Corporation) | 0.5g/L |
| Lysolecithin (made by Sigma Chemical Co.) | 0.2g/L |

| Reagent 2 | |
|---|---|
| Anti-C-reactive protein antibody sensitized latex (prepared by reference 3) | 1g/L |

### Comparison B-5

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |

| Reagent 2 | |
|---|---|
| Anti-C-reactive protein antibody sensitized latex (prepared by reference 3) | 1g/L |

### Example B-4

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Octadecyltrimethylammonium chloride (NISSAN CationAB, made by NOF Corporation) | 0.5g/L |
| Phosphorylcholine oleyloxyethyl ester (made by Sigma Chemical Co.) | 0.3g/L |

| Reagent 2 | |
|---|---|
| Anti-C-reactive protein antibody sensitized latex (prepared by reference 1) | 1g/L |

### Example B-5

The following measuring reagents of C-reactive protein are prepared.

| Reagent 1 | |
|---|---|
| Glycine buffer (made by Kanto Chemical Co., Inc.) | 100mmol/L |
| Calcium chloride (made by Kanto Chemical Co., Inc.) | 5mmol/L |
| Octadecyltrimethylammonium chloride (NISSAN CationPB, made by NOF Corporation) | 0.5g/L |
| Sphingosyl phosphorylcholine (made by Sigma Chemical Co.) | 0.3g/L |

| Reagent 2 | |
|---|---|
| Anti-C-reactive protein antibody sensitized latex (prepared by reference 1) | 1g/L |

### Example B-6

The concentration of C-reactive protein in serum solutions containing C-reactive protein of various kinds of concentration (the concentration of C-reactive protein: 0∼100mg/dL) were measured with the measuring reagents of C-reactive protein prepared by examples B-1∼B-2 and comparisons B-1∼B-4. 147µL of the reagent 1, 150µL of the reagent 2 and 3µL of the standard serum containing C-reactive protein were mixed at 37°C, then the variations of integrating sphere turbidity between 72 seconds and 216 seconds (23∼31 points) were measured by EL-1060 (made by Kyowa Medex Co., Ltd.), an equipment for measuring integrating sphere turbidity.

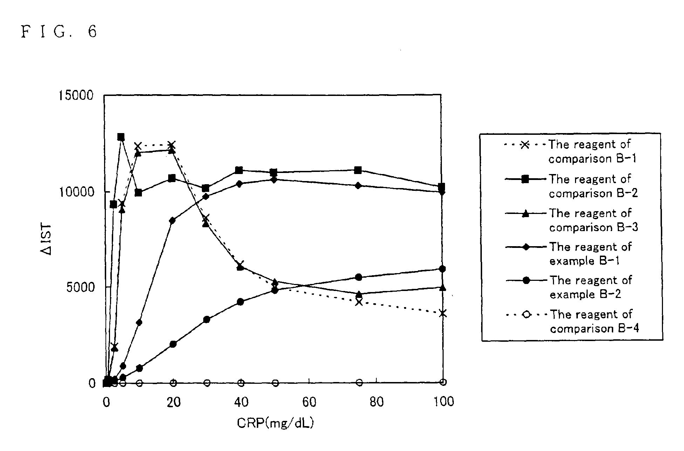

The results are shown in Fig. 6. In Fig. 6, CRP of the horizontal axis stands for the concentration of C-reactive protein, and ΔIST value of the vertical axis stands for the variations of integrating sphere turbidity. In the measurement with the reagent of comparison B-1 (-×-), prozone occurred when the concentration of C-reactive protein was 10mg/dL or over. In the measurement with the reagent of comparison B-2 (-■-), prozone tended to be avoided, but the improvement of measurement range was not observed. In the measurement with the reagent of comparison B-3 (-▲-), no effect of avoiding prozone was observed. In the measurement with the reagent of comparison B-4 (-○-), ΔIST value defied detection. Whereas in the measurement with the reagent of example B-1 (-◆-), the measurement range was greatly improved. In the measurement with the reagent of example B-2 (-●-), prozone could be avoided at least up to the concentration of 100mg/dL, and the quantitativity improved greatly.

### Example B-7

The concentration of C-reactive protein in the serum solutions containing C-reactive protein of various kinds of concentration (the concentration of C-reactive protein: 0∼100mg/dL) were measured with the measuring reagents of C-reactive protein prepared by example B-3 and comparison B-5. 225µL of the reagent 1, 75µL of the reagent 2 and 3µL of the standard serum containing C-reactive protein were mixed at 37°C, then the variations of absorbance (wave-length 570nm) between 19 points and 27 points were measured by the absorption spectro photometric autoanalyzer 7070 (made by Hitachi Ltd.).

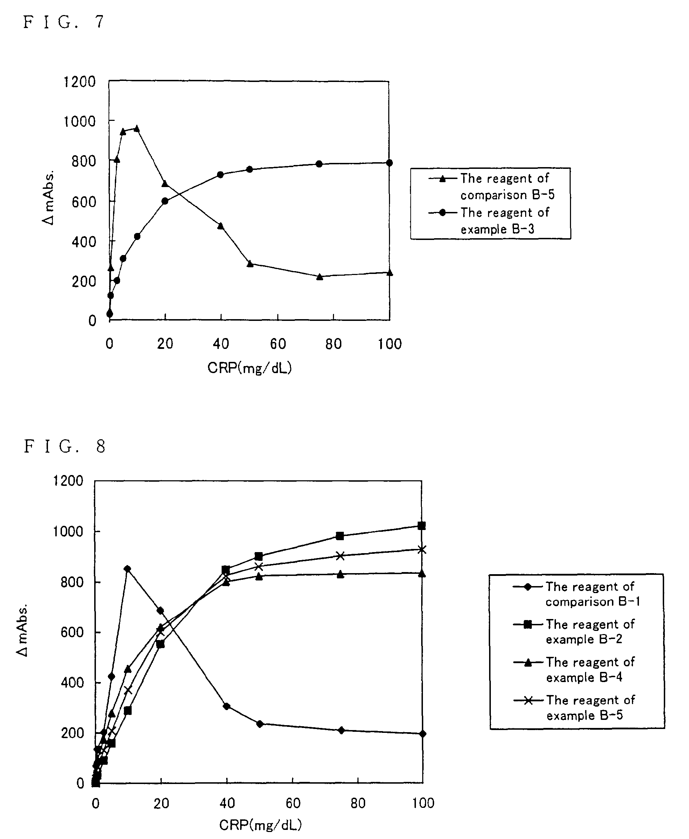

The results are shown in Fig. 7. In Fig. 7, CRP of the horizontal axis stands for the concentration of C-reactive protein, and ΔmAbs. (570nm) of the vertical axis stands for the variations of absorbance of 570nm. In the measurement with the reagent of comparison B-5 (-▲-), prozone occurred and the variations of absorbance lowered when the concentration of C-reactive protein was 5mg/dL or over. Whereas in the measurement with the reagent of example B-3 (-●-), at least up to the concentration of 100mg/dL, the variations of absorbance did not lower, prozone could be avoided, and the quantitativity improved greatly.

### Example B-8

The concentration of C-reactive protein in the serum solutions containing C-reactive protein of various kinds of concentration (the concentration of C-reactive protein: 0∼100mg/dL) were measured with the measuring reagents of C-reactive protein prepared by example B-2, example B-4, example B-5 and comparison B-1. 225µL of the reagent 1, 75µL of the reagent 2 and 3µL of the standard serum containing C-reactive protein were mixed at 37°C, then the variations of absorbance (wave-length 570nm) between 19 points and 27 points were measured by the absorption spectro photometric autoanalyzer 7070 (made by Hitachi Ltd.).

The results are shown in Fig. 8. In Fig. 8, CRP of the horizontal axis stands for the concentration of C-reactive protein, and ΔmAbs, of the vertical axis stands for the variations of absorbance. In the measurement with the reagent of comparison B-1 (-◆-), when the concentration of C-reactive protein was 10mg/dL or over, prozone occurred and the variations of absorbance lowered. Whereas in the measurement with the reagent of example B-2 (-■-), with the reagent of example B-4 (-▲-), and with the reagent of example B-5(-×-), at least up to the concentration of 100mg/dL, the variations of absorbance did not lower, prozone could be avoided, and the quantitativity improved greatly.

### Reference 1 (Preparation of latex reagent for measuring CRP)

0.8mL of polystyrene latex solution with an average particle diameter of 130nm (made by Kyowa Medex Co., Ltd., 100mg/mL) and 1mL of goat anti-C-reactive protein polyclonal antibody solution (made by Oriental Yeast Co., Ltd., 10mg/mL, 50mM phosphoric acid-NaCl 150mM pH7.2) were mixed and stirred for 30 minutes at 37°C. Then 1mL of bovine serum albumin (BSA, made by Sigma Chemical Co.) solution (20mg/mL, 50mM phosphoric acid-NaCl 150mM pH7.2) was added and the mixture was stirred for 2 hours at 37°C and had blocking treatment. Then the latex was sedimented in pellet-like shape by centrifugation (50,00 revolutions, for 30 minutes), and after removing the supernatant, 10mL of 50mM imidazole-hydrochloride solution (pH7.8) containing 3mg/mL of BSA was added and the mixture was stirred enough, then had dispersion treatment by ultrasonic crusher. The excess antibody solution was removed by doing above-mentioned operation three times. Finally, the concentration of latex was arranged to be 1mg/mL, and the latex reagent of measuring C-reactive protein was prepared. This reagent was stored at 4°C.

### Reference 2

Control reagent containing no anti-C-reactive protein antibody was prepared by the method in which goat anti-C-reactive protein polyclonal antibody solution was not used, but otherwise it was the same method as reference 1.

### Reference 3

The latex reagent of measuring C-reactive protein was prepared by the method in which 0.8mL of polystyrene latex solution with an average particle diameter of 220nm (made by Kyowa Medex Co., Ltd., 100mg/mL) was used, but otherwise it was the same method as reference 1. The final concentration was arranged to be 1mg/mL as in reference 1, and this reagent was stored at 4°C.

### Reference 4

37.2g of 2-methacryloyloxyethyl phosphorylcholine (made by NOF Corporation), 12.8g of 2-hydoroxy-3-methacryloyloxypropyltrimethylammonium chloride (made by NOF Corporation) 0.3g of a polymerization initiator, 2,2'-azobis (2-methylpropionoamidine) dihydrochloride ("V-50" made by Wako Pure Chemical Industries, Ltd.), 150g of water as a polymerization medium were used and warmed to 70°C for 4 hours in order to cause polymerization reaction. After the polymerization reaction completed, the reaction liquid was slowly dropped into 1.5L of acetone and polymerized substance was precipitated. The precipitate was separated by filtration, then dried and dissolved into distilled water so that the concentration would be 5.0 weight %. The phosphoric acid buffer of the polymerized substance was analyzed by gel permeation chromatography (GPC), and the weight average molecular weight was found to be 37,000 with poly(ethylene oxide) standards. This compound contains a phosphorylcholine group and a cationic group with the molar ratio of 7:3.

### Reference 5

45.9g of 2-methacryloyloxyethyl phosphorylcholine (made by NOF Corporation), 4.1g of 2-hydoroxy-3-methacryloyloxypropyltrimethylammonium chloride (made by NOF Corporation), 0.3g of a polymerization initiator, 2,2'-azobis (2-methylpropiono amidine) dihydrochloride ("V-50" made by Wako Pure Chemical Industries, Ltd.), 150g of water as a polymerization medium were used and warmed to 70°C for 4 hours in order to cause polymerization reaction. After the polymerization reaction completed, the reaction liquid was slowly dropped into 1.5L of acetone and polymerized substance was precipitated. The precipitate was separated by filtration, then dried and dissolved into distilled water so that the concentration would be 5.0 weight %. The phosphoric acid buffer of the polymerized substance was analyzed by gel permeation chromatography (GPC), and the weight average molecular weight was found to be 33,000 with poly(ethylene oxide) standards. This compound contains a phosphorylcholine group and a cationic group with the molar ratio of 9:1.

### Reference 6

50.0g of 2-methacryloyloxyethyl phosphorylcholine (made by NOF Corporation), 0.24g of a polymerization initiator, azobis isobutyronitrile ("AIBN" made by Wako Pure Chemical Industries, Ltd.), 100g of ethanol as a polymerization medium were used and warmed to 70°C for 4 hours in order to cause polymerization reaction. After the polymerization reaction completed, the reaction liquid was slowly dropped into 1.5L of acetone and polymerized substance was precipitated. The precipitate was separated by filtration, then dried and dissolved into distilled water so that the concentration would be 5.0 weight %. The phosphoric acid buffer of the polymerized substance was analyzed by gel permeation chromatography (GPC), and the weight average molecular weight was found to be 108,000 with poly(ethylene oxide) standards.

### Reference 7

35.7g of 2-methacryloyloxyethyl phosphorylcholine (made by NOF Corporation), 4.3g of butylmethacrylate (made by Wako Pure Chemical Industries, Ltd.), 0.82g of a polymerization initiator, azobis isobutyronitrile ("AIBN" made by Wako Pure Chemical Industries, Ltd.), 160g of ethanol as a polymerization medium were used and warmed to 70°C for 4 hours in order to cause polymerization reaction. After the polymerization reaction completed, the reaction liquid was slowly dropped into 1.5L of acetone and polymerized substance was precipitated. The precipitate was separated by filtration, then dried and dissolved into distilled water so that the concentration would be 5.0 weight %. The phosphoric acid buffer of the polymerized substance was analyzed by gel permeation chromatography (GPC), and the weight average molecular weight was found to be 87,000 with poly(ethylene oxide) standards. This compound contains a phosphorylcholine group and a butyl group with the molar ratio of 8:2.

### Reference 8

74.3g of 2-methacryloyloxyethyl phosphorylcholine (made by NOF Corporation), 25.7g of 2-hydoroxy-3-methacryloyloxypropyltrimethylammonium chloride (made by NOF Corporation), 0.45g of a polymerization initiator, 2,2'-azobis (2-methylpropiono amidine) dihydrochloride ("V-50" made by Wako Pure Chemical Industries, Ltd.), 900g of water as a polymerization medium were used and warmed to 60°C for 4 hours in order to cause polymerization reaction. After the polymerization reaction completed, the reaction liquid was slowly dropped into 1.5L of acetone and polymerized substance was precipitated. The precipitate was separated by filtration, then dried and dissolved into distilled water so that the concentration would be 5.0 weight %. The phosphoric acid buffer of the polymerized substance was analyzed by gel permeation chromatography (GPC), and the weight average molecular weight was found to be 13,000 with poly(ethylene oxide) standards. This compound contains a phosphorylcholine group and a cationic group with the molar ratio of 7:3. Next, the centrifugation (3,000×g, 30 minutes) was conducted with a centrifugal filtration tube (limited molecular weight by ultrafiltration: 10,000, made by Millipore Corp.) and ingredients with the molecular weight of 10,000 and under, obtained as filtrate, was recovered.

### Industrial Applicability:

By using the reagent of C-reactive protein of the present invention, it becomes possible to avoid prozone phenomenon and to broaden the range of measurement upon measuring C-reactive protein. This enables us to determine the subject substance without dilution even when it contains high concentration of C-reactive protein.

## Claims

1. A measuring method of C-reactive protein characterized in measuring C-reactive protein with a compound having a phosphorylcholine group and a cationic group excluding a phosphorylcholine group, and an antibody to C-reactive protein, or with a surface active agent having a phosphorylcholine group, a surface active agent having a cationic group excluding a phosphorylcholine group, and an antibody to C-reactive protein.

2. A measuring method of C-reactive protein according to claim 1, wherein a cationic group in a compound having a phosphorylcholine group and a cationic group excluding a phosphorylcholine group is a group shown by the general formula (I) 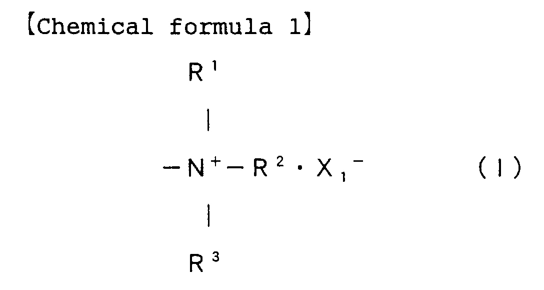 [in the formula (I), where R¹, R² and R³ are same or different from one another, and each of them stands for a hydrogen atom, substituted or non-substituted alkyl, or substituted or non-substituted alkenyl, and X₁⁻ stands for an inorganic anion or an organic anion].

3. A measuring method of C-reactive protein according to claim 1 or 2, wherein a compound having a phosphorylcholine group and a cationic group excluding a phosphorylcholine group is a copolymer created by combining a monomer having a phosphorylcholine group and a monomer having a cationic group.

4. A measuring method of C-reactive protein according to claim 3, wherein a monomer having a phosphorylcholine group and a monomer having a cationic group are a monomer having a phosphorylcholine group and a vinyl group, and a monomer having a cationic group and a vinyl group respectively.

5. A measuring method of C-reactive protein according to claim 4, wherein a monomer having a phosphorylcholine group and a vinyl group, and a monomer having a cationic group and a vinyl group are 2-methacryloyloxyethyl phosphorylcholine and 2-hydroxy-3-methacryloyloxypropyltrimethylammonium chloride respectively.

6. A measuring method of C-reactive protein according to claim 1, wherein a surface active agent having a phosphorylcholine group is a compound shown by the general formula (II) 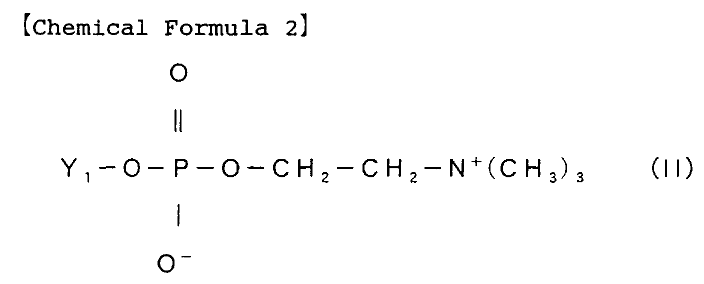 [in the formula (II), Y₁ stands for a hydrophobic group].

7. A measuring method of C-reactive protein according to claim 6, wherein a compound shown by the formula (II) is one or more kinds of compounds selected from lysophosphatidylcholine caproyl, lysophosphatidylcholine myristoyl, lysophosphatidylcholine palmitoyl, lysophosphatidylcholine stearoyl, lysophosphatidylcholine derived from soybeans, phosphatidylcholine dibutyloyl, phosphatidylcholine dicaproyl, phosphorylcholine oleyloxyethyl ester and sphingosyl phosphorylcholine.

8. A measuring method of C-reactive protein according to any one of claims 1, 6 or 7, wherein a surface active agent having a cationic group is a surface active agent of ammonium salt.

9. A measuring method of C-reactive protein according to claim 8, wherein a surface active agent of ammonium salt is a compound shown by the general formula (III) 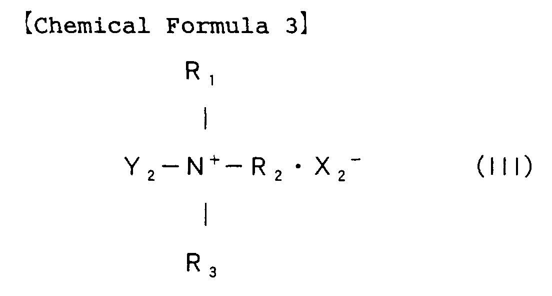 [in the formula (III), Y2 stands for a hydrophobic group, R₁, R₂ and R₃ are same or different from one another, and each of them stands for a hydrogen atom, substituted or non-substituted alkyl, or substituted or non-substituted alkenyl, and X₂⁻ stands for an inorganic anion or an organic anion].

10. A measuring method of C-reactive protein according to claim 9, wherein a compound shown by the formula (III) is one or more kinds of compounds selected from octadecyltrimethylammonium chloride, hexadecyltrimethylammonium chloride, tetradecyltrimethylammonium chloride and dodecyltrimethylammonium chloride.

11. A measuring method of C-reactive protein according to any one of claims 1∼10, wherein an antibody to C-reactive protein is carried by a water-insoluble carrier.

12. A measuring method of C-reactive protein according to claim 11, wherein an insoluble carrier is latex made from polystyrene.

13. A measuring reagent of C-reactive protein characterized in containing a compound having a phosphorylcholine group and a cationic group excluding a phosphorylcholine group, and an antibody to C-reactive protein, or containing a surface active agent having a phosphorylcholine group, a surface active agent having a cationic group excluding a phosphorylcholine group, and an antibody to C-reactive protein.

14. A measuring reagent of C-reactive protein according to claim 13, wherein a cationic group in a compound having a phosphorylcholine group and a cationic group excluding a phosphorylcholine group is shown by the general formula (I) 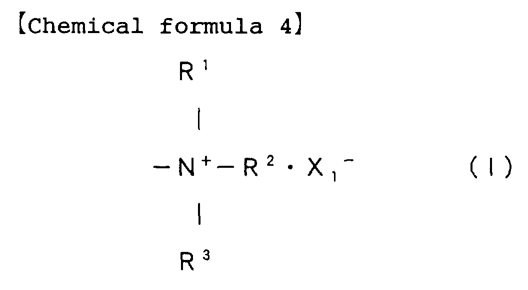 [in the formula (I), where R¹, R² and R³ are same or different from one another, each of them stands for a hydrogen atom, substituted or non-substituted alkyl, or substituted or non-substituted alkenyl, and X₁⁻ stands for an inorganic anion or an organic anion].

15. A measuring reagent of C-reactive protein according to claims 13 or 14, wherein a compound having a phosphorylcholine group and a cationic group excluding a phosphorylcholine group is a copolymer created by combining a monomer having a phosphorylcholine group and a monomer having a cationic group.

16. A measuring reagent of C-reactive protein according to claim 15, wherein a monomer having a phosphorylcholine group and a monomer having a cationic group are a monomer having a phosphorylcholine group and a vinyl group, and a monomer having a cationic group and a vinyl group respectively.

17. A measuring reagent of C-reactive protein according to claim 16, wherein a monomer having a phosphorylcholine group and a vinyl group, and a monomer having a cationic group and a vinyl group are 2-methacryloyloxyethyl phosphorylcholine and 2-hydroxy-3-methacryloyloxypropyltrimethylammonium chloride respectively.

18. A measuring reagent of C-reactive protein according to claim 13, wherein a surface active agent having a phosphorylcholine group is a compound shown by the general formula (II) 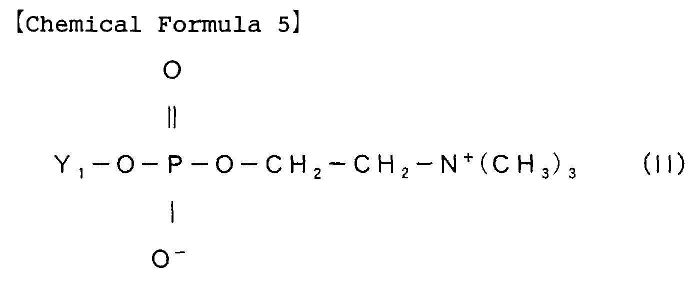 [Y₁ in the formula (II) stands for a hydrophobic group].

19. A measuring reagent of C-reactive protein according to claim 18, wherein a compound shown by the formula (II) is one or more kinds of compounds selected from lysophosphatidylcholine caproyl, lysophosphatidylcholine myristoyl, lysophosphatidylcholine palmitoyl, lysophosphatidylcholine stearoyl, lysophosphatidylcholine derived from soybeans, phosphatidylcholine dibutyloyl, phosphatidylcholine dicaproyl, phosphorylcholine oleyloxyethyl ester and sphingosyl phosphorylcholine.

20. A measuring reagent of C-reactive protein according to any one of claims 13, 18 or 19, wherein a surface active agent having a cationic group is a surface active agent of ammonium salt.

21. A measuring reagent of C-reactive protein according to claim 20, wherein a surface active agent of ammonium salt is a compound shown by the general formula (III) 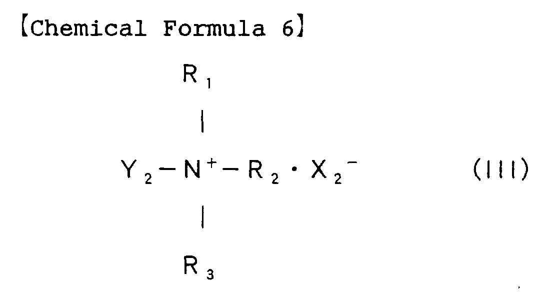 [in the formula (III), Y2 stands for a hydrophobic group, R₁, R₂ and R₃ are same or different from one another, and each of them stands for a hydrogen atom, substituted or non-substituted alkyl, or substituted or non-substituted alkenyl, and X₂⁻ stands for an inorganic anion or an organic anion].

22. A measuring reagent of C-reactive protein according to claim 21, wherein a compound shown by the formula (III) is one or more kinds of compounds selected from octadecyltrimethylammonium chloride, hexadecyltrimethylammonium chloride, tetradecyltrimethylammonium chloride and dodecyltrimethylammonium chloride.

23. A measuring reagent of C-reactive protein according to any one of claims 13∼22, wherein an antibody to C-reactive protein is carried by a water-insoluble carrier.

24. A measuring reagent of C-reactive protein according to claim 23, wherein an insoluble carrier is latex made from polystyrene.
